(19) Europäisches Patentamt
European Patent Office
Office européen des brevets

(11) **EP 1 656 343 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**11.10.2017 Patentblatt 2017/41**

(45) Hinweis auf die Patenterteilung:
**01.10.2014 Patentblatt 2014/40**

(21) Anmeldenummer: **04763738.4**

(22) Anmeldetag: **03.08.2004**

(51) Int Cl.:
*C07C 319/08* (2006.01)   *B01J 23/30* (2006.01)
*B01J 23/04* (2006.01)

(86) Internationale Anmeldenummer:
**PCT/EP2004/008680**

(87) Internationale Veröffentlichungsnummer:
**WO 2005/021491 (10.03.2005 Gazette 2005/10)**

(54) **KATALYSATOR ZUR SYNTHESE VON ALKYLMERCAPTAN UND VERFAHREN ZU SEINER HERSTELLUNG**

CATALYST FOR SYNTHESIZING ALKANETHIOL AND METHOD FOR THE PRODUCTION THEREOF

CATALYSEUR POUR LA SYNTHESE D'ALCANETHIOL ET PROCEDE DE PRODUCTION DUDIT CATALYSEUR

(84) Benannte Vertragsstaaten:
**AT BE BG CH CY CZ DE DK EE ES FI FR GB GR HU IE IT LI LU MC NL PL PT RO SE SI SK TR**

(30) Priorität: **23.08.2003 DE 10338887**

(43) Veröffentlichungstag der Anmeldung:
**17.05.2006 Patentblatt 2006/20**

(73) Patentinhaber: **Evonik Degussa GmbH**
**45128 Essen (DE)**

(72) Erfinder:
• **REDLINGSHÖFER, Hubert**
**91481 Münchsteinach (DE)**
• **WECKBECKER, Christoph**
**63584 Gründau-Lieblos (DE)**
• **DÖRFLEIN, Andreas**
**63538 Grosskrotzenburg (DE)**
• **RÜCKRIEGEL, Michael**
**63599 Biebergemünd (DE)**

(56) Entgegenhaltungen:
EP-A- 0 832 687   EP-A2- 0 832 687
US-A- 2 820 062   US-A- 5 852 219

• **LARUELLE ET AL.: "High-Energy Milling of WO3 oxides: Amorphization and Reaction with Cs2CO3" JOURNAL OF SOLID STATE CHEMISTRY, 1994, Seiten 172-177, XP002304455**

• **T. KUDO ET AL.: "Hexagonal and pyrochlore-type cesium tungstates synthesized from cesium peroxo-polytungstate and their intercalation chemistry" SOLID STATE IONICS, 1994, Seiten 204-208, XP002304456**
• **QIMING ZHONG ET AL: "ELECTROCHROMIC PROPERTIES OF CESIUM TUNGSTATE WITH PYROCHLORE STRUCTURE" THIN SOLID FILMS, ELSEVIER-SEQUOIA S.A. LAUSANNE, CH, Bd. 205, Nr. 1, 30. Oktober 1991 (1991-10-30), Seiten 85-88, XP000261746 ISSN: 0040-6090**
• **MASHKINA A V ET AL: "ACTIVITY OF TUNGSTATE CATALYSTS IN THE SYNTHESIS OF METHYL - MERCAPTANE FROM METHANOL AND HYDROGEN SULFIDE" REACTION KINETICS AND CATALYSIS LETTERS, ELSEVIER, AMSTERDAM, NL, Bd. 36, Nr. 1, 1988, Seiten 159-164, XP002063476 ISSN: 0133-1736 in der Anmeldung erwähnt**
• **MASHKINA A.V. ET AL: 'Activity of tungstate catalyst in the synthesis of methyl - Mercaptane from methanol and hydrogen sulfide' REACTION KINETICS AND CATALYSIS LETTERS Bd. BD. 36, Nr. 1, 1988, Seiten 159 - 164**
• **BRÜDGAM I. ET AL: 'Two new isopolyoxotungstates(VI) with the empirical composition Cs2W2O7.2H2O and Na2W2O7.H2O: an icosatetratungstate and a polymeric compound' ANGEW. CHEM. INT. ED. Bd. 37, Nr. 19, 1988, Seiten 2668 - 2671**
• **IVANNIKOVA N.V. ET AL: 'Behavior of cesium lantahnum tungstate (CsLa(WO4)2) during heating' J. OF INORG. CHEM. Bd. 32, Nr. 1, 1987, Seiten 180 - 183**

EP 1 656 343 B2

## Beschreibung

Die vorliegende Erfindung betrifft einen oxidischen, Cäsium und Wolfram enthaltenden Katalysator zur Synthese von Alkylmercaptanen aus Alkanolen und Schwefelwasserstoff, sowie ein Verfahren zur Herstellung dieses Katalysators.

Insbesondere Methylmercaptan ist ein industriell wichtiges Zwischenprodukt zum Beispiel für die Synthese von Methionin sowie für die Synthese von Dimethylsulfoxid und Dimethylsulfon. Es wird heute überwiegend aus Methanol und Schwefelwasserstoff durch Reaktion an einem Katalysator aus Aluminiumoxid hergestellt. Die Synthese des Methylmercaptans erfolgt gewöhnlich in der Gasphase bei Temperaturen zwischen 300 und 500°C und bei Drücken zwischen 1 und 25 bar.

Das Reaktionsgemisch enthält neben dem gebildeten Methylmercaptan die nicht umgesetzten Ausgangsstoffe und Nebenprodukte, wie zum Beispiel Dimethylsulfid und Dimethylether sowie die im Sinne der Reaktion inerten Gase, wie zum Beispiel Methan, Kohlenmonoxid, Wasserstoff und Stickstoff. Aus diesem Reaktionsgemisch wird das gebildete Methylmercaptan abgetrennt.

Für die Wirtschaftlichkeit des Verfahrens wird eine möglichst hohe Selektivität bei der katalytischen Reaktion von Methanol und Schwefelwasserstoff zu Methylmercaptan gefordert, um den Aufwand bei der Abtrennung des gebildeten Methylmercaptans vom Reaktionsgemisch so gering wie möglich zu halten. Hier stellt insbesondere der Energieaufwand zur Kühlung des Reaktionsgasgemisches zur Kondensation des Methylmercaptans einen großen Kostenfaktor dar.

Zur Erhöhung von Aktivität und Selektivität wird Aluminiumoxid als Träger üblicherweise mit Kaliumwolframat oder Cäsiumwolframat versetzt. Dabei wird das Wolframat gewöhnlich in Mengen bis 25 Gew.-%, bezogen auf das Gesamtgewicht des Katalysators, eingesetzt. Eine Verbesserung von Aktivität und Selektivität erhält man auch durch Erhöhung des Molverhältnisses von Schwefelwasserstoff zu Methanol. Üblicherweise werden Molverhältnisse zwischen 1 und 10 angewendet.

Ein hohes Molverhältnis bedeutet allerdings auch einen hohen Überschuss des Schwefelwasserstoffes im Reaktionsgemisch und somit die Notwendigkeit, große Gasmengen im Kreis zu führen. Zur Verminderung des hierfür benötigten Energieaufwandes sollte daher das Verhältnis von Schwefelwasserstoff zu Methanol nur wenig von 1 abweichen.

US 2,820,062 betrifft ein Verfahren zur Herstellung von organischen Thiolen, bei welchem ein Katalysator aus aktivem Aluminiumoxid verwendet wird, der mit Kaliumwolframat in einer Menge von 1,5 bis 15 Gew.-%, bezogen auf das Gewicht des Katalysators, versetzt ist. Mit diesem Katalysator werden gute Aktivitäten und Selektivitäten bei Reaktionstemperaturen von 400°C und Molverhältnissen von 2 erzielt. Diese US-Patentschrift nennt verschiedene Möglichkeiten zur Einbringung des Kaliumwolframats in das Aluminiumoxid. So sollen Imprägnierverfahren, Copräzipitationen und reine Mischungen anwendbar sein. Der eigentlichen Herstellung des Katalysators wird wenig Bedeutung für die Wirtschaftlichkeit des Syntheseverfahrens von Methylmercaptan eingeräumt.

In der EP 0 832 687 B1 werden die Vorteile der Verwendung von Cäsiumwolframat ($Cs_2WO_4$) anstelle von Kaliumwolframat ($K_2WO_4$) als Promotor beschrieben. So kann durch Einsatz von Cäsiumwolframat eine gesteigerte Aktivität bei gleichzeitig guter Selektivität erreicht werden.

Durch Erhöhung der Cäsiumwolframat-Konzentration auf bis zu 40 Gew.-% kann die Selektivität zu Methylmercaptan auf bis zu 92% gesteigert werden, ohne dass die Aktivität unverhältnismäßig verschlechtert wird.

Wolframbronzen der allgemeinen Formel $Cs_xWO_3$ mit einem niedrigeren Alkali/Wolframverhältnis werden in den wissenschaftlichen Veröffentlichungen S. Laruelle et al., Journal of Solid State Chemistry 111, 172-177 (1994), T. Kudo, Solid State Ionics 72. 204-208 (1994) und Q. Zhong et al., Thin Solid Films 205. 85-88 (1991) beschrieben.

Nach allgemeiner Ansicht erzielt man die beste Selektivität mit Katalysatoren, bei denen das Alkali/Wolframverhältnis gleich 2:1 ist (A.V. Mashkina et al., React. Kinet. Catal. Lett., Vol. 36, No. 1, 159-164 (1988)).

Aufgabe der vorliegenden Erfindung ist es, einen Katalysator und ein Verfahren zu seiner Herstellung anzugeben, welcher sich bei niedrigen Molverhältnissen von Schwefelwasserstoff zu Methanol durch verbesserte Aktivität und Selektivität gegenüber den bekannten Katalysatoren auszeichnet und somit zu einer besseren Wirtschaftlichkeit des Verfahrens führt.

Diese Aufgabe wird durch die Bereitstellung eines Trägerkatalysators gelöst, enthaltend eine katalytisch aktive oxidische Zusammensetzung der allgemeinen Formel $Cs_xWO_y$, in der x:< 2 bis 0,8 und y:3,4 bis < 4 bedeuten, wobei der Trägerkatalysator die oxidische Zusammensetzung in einer Menge von 15 bis 45 Gew.-% enthält, und wobei ein Trägerkörper oder ein pulverförmiger Träger mit einer wässrigen Imprägnierlösung aus in Wasser löslichen Cs- und Wolframverbindungen (Promotorlösung) imprägniert und abschließend kalziniert wird.

Der Katalysator enthält diese Zusammensetzung insbesondere in einer Menge von 20 bis 36 Gew.-%, bevorzugt >25 bis 36 Gew.-%.

Gegenstand der Erfindung ist außerdem ein Schalenkatalysator, bei dem ein Trägerkern mit einem pulverförmigen Trägerkatalysator gemäß der vorliegenden Erfindung umhüllt ist.

Ein weiterer Gegenstand der Erfindung ist ein Vollkatalysator bei dem ein pulverförmiger Trägerkatalysator gemäß der vorliegenden Erfindung verformt wird.

Bevorzugt beläuft sich im erfindungsgemäßen Vollkatalysator das Verhältnis von Cäsium zu Wolfram auf 1,9:1 bis 1:1.

Die Promotorlösung kann zur Herstellung des Katalysators in Form eines Trägerkatalysators direkt auf einen Trägerkörper imprägniert werden. Bei der Herstellung von Katalysatoren in Form von Extrudaten oder Preßlingen wird der pulverförmige Träger mit der Promotorlösung imprägniert und kalziniert und die erhaltene Zwischenstufe anschließend verformt. Wird ein Schalenkatalysator hergestellt, so wird der pulverförmige Träger mit Promotorlösung imprägniert und kalziniert und die entstehende Zwischenstufe dann auf einen inerten Trägerkern in Form einer Schale aufgebracht.

Die Trägermaterialien können in einzelnen Fällen auch zur katalytischen Aktivität beitragen.

Das Cs/W-Verhältnis beläuft sich bevorzugt auf < 1,9:1 bis 1:1. Damit enthalten die erfindungsgemäßen Katalysatoren für die Umsetzung von Alkanolen mit Schwefelwasserstoff zu Alkylmercaptanen einen im Vergleich zum mit Cäsiumwolframat ($Cs_2WO_4$) imprägnierten Katalysator aus dem Stand der Technik überstöchiometrischen Anteil an Wolfram.

Es zeigt sich, dass dieser höhere Anteil auf dem bevorzugt eingesetzten Aluminiumoxid im Vergleich zu dem im Stand der Technik ausschließlich verwendeten stöchiometrischen Erdalkali- oder Alkaliwolframat dem Katalysator eine verbesserte Aktivität bei gleichzeitig verbesserter Selektivität verleiht. Während die Erhöhung der Konzentration von Cäsiumwolframat ($Cs_2WO_4$) auf dem Katalysator lediglich eine Steigerung der Selektivität bei gleichzeitig geringerer Aktivität bewirkt, zeigt sich bei ausschließlicher Erhöhung des Wolframgehalts und unverändertem Cäsiumgehalt in unerwarteter Weise eine weitere Steigerung der Selektivität bei gleichzeitig gesteigerter Aktivität.

Erfindungsgemäß kann bei sehr hohen Beladungen mit dem Promotor eine ausgezeichnete Selektivität erreicht werden, ohne dass die Aktivität des Katalysators, wie aus dem Stand der Technik bekannt, abnimmt. Weiterhin wurde gefunden, dass über das Alkali-Wolfram-Verhältniss die Aktivität und Selektivität des Katalysators gezielt eingestellt werden kann. Der Katalysator wird in Form eines Trägerkatalysators, bei dem die Oberfläche mit der Promotorlösung imprägniert und kalziniert wird, oder eines Schalenkatalysators, bei dem ein Kern mit einem mit der Promotorlösung imprägnierten und kalzinierten Trägermaterial umgeben ist, eingesetzt. Weiterhin können Extrudate oder Preßlinge, bei denen das pulverförmige Trägermaterial vor der Verformung mit der Promotorlösung imprägniert und kalziniert wird, verwendet werden (Vollkatalysator).

Als Trägermaterialien für diesen Katalysator werden die bekannten oxidischen anorganischen Verbindungen wie zum Beispiel $SiO_2$, $TiO_2$, $ZrO_2$ und bevorzugt sogenanntes aktives Aluminiumoxid eingesetzt. Dieses Material weist hohe spezifische Oberflächen zwischen etwa 10 und 400 m$^2$/g auf und besteht hauptsächlich aus Oxiden der Übergangsreihe der kristallographischen Phasen des Aluminiumoxids (siehe zum Beispiel Ullmann's Encyclopedia of Industrial Chemistry von 1985, Vol. A1, Seiten 561-562). Zu diesen Übergangsoxiden gehören $\gamma$-, $\delta$-, $\eta$-, $\kappa$-, $\chi$- und $\theta$-Aluminiumoxid. Alle diese kristallographischen Phasen gehen bei Erhitzung des Aluminiumoxids auf Temperaturen über 1100°C in das thermisch stabile $\alpha$-Aluminiumoxid über. Aktives Aluminiumoxid wird kommerziell für katalytische Anwendungen in verschiedenen Qualitäten und Lieferformen angeboten.

Besonders geeignet für die Herstellung von Trägerkatalysatoren sind Formkörper aus granuliertem oder stranggepresstem Aluminiumoxid mit Korndurchmessern von 1 bis 5 mm, einer spezifischen Oberfläche von 180 bis 400 m$^2$/g, einem Gesamtporenvolumen zwischen 0,3 und 1,2 ml/g sowie einer Schüttdichte von 300 bis 900 g/l. Für die Zwecke der Erfindung wird bevorzugt Aluminiumoxid mit mehr als 200 m$^2$/g spezifischer Oberfläche verwendet, da die katalytische Aktivität des fertigen Katalysators mit steigender Oberfläche des Aluminiumoxids leicht ansteigt. Dieses Material wird in Pulverform bevorzugt für die Herstellung der Schalenkatalysatoren, Extrudate oder Preßlinge eingesetzt.

Die wässrige Imprägnierlösung zur Aufbringung des Promotors kann in einfacher Weise aus in Wasser löslichen Cs- und Wolframverbindungen, insbesondere Wolframsäure ($H_2WO_4$) und Cäsiumhydroxid ($Cs(OH)\cdot H_2O$) hergestellt werden. Hierzu wird z.B. Wolframsäure in Wasser suspendiert und unter Zugabe einer Base und Erwärmen aufgelöst. Cäsiumhydroxid oder ein anderes Cäsiumsalz wird ebenfalls in Wasser aufgelöst und mit der Lösung der Wolframsäure vereinigt (Promotorlösung). Bevorzugt eingesetzt werden Cäsiumsalze, deren Anionen sich durch Wärmebehandlung rückstandslos austreiben lassen wie z.B. Nitrate, Formiate, Oxalate, Acetate oder Carbonate. Zur Stabilisierung dieser Lösung mit einem pH von 8 bis 14 eignen sich anorganische und auch organische Basen.

Bevorzugt werden solche Basen eingesetzt, die sich durch eine abschließende Wärmebehandlung des nach der Imprägnierung erhaltenen Katalysators rückstandslos austreiben lassen. Zu diesen Basen gehören bevorzugt Ammoniumhydroxid und organische Basen, insbesondere Amine. Gegenüber dem Stand der Technik wird das molare Verhältnis von Cs und W beim Ansetzen der wässrigen Imprägnierlösung derart gewählt, dass im Unterschied zu Cäsiumwolframat ($Cs_2WO_4$) mit einem Cs- W-Verhältnis von 2 zu 1 ein höherer Anteil an Wolfram, d.h. ein Cs zu W-Verhältnis kleiner als 2 zu 1, insbesondere < 1,9:1 bis 0,8:1 vorliegt. Dies führt im Vergleich zu den bekannten Katalysatoren zu einer deutlich erhöhten Aktivität und Selektivität der erfindungsgemäßen Katalysatoren, insbesondere bei niedrigen Verhältnissen von Schwefelwasserstoff und Methanol im Reaktionsgas.

Für das Aufbringen der Promotorlösung können verschiedene Imprägniertechniken wie das Tauchimprägnieren, Sprühimprägnieren, Vakuumimprägnieren und die Porenvolumenimprägnierung eingesetzt werden, wobei die Imprägnierung auch mehrfach erfolgen kann. Bei Formkörpern muss das gewählte Imprägnierverfahren es ermöglichen, die gewünschte Beladungsmenge des Promotors mit guter Gleichmäßigkeit über den gesamten Querschnitt aufzubringen.

Bevorzugt wird die Promotorlösung durch Sprüh- oder Vakuumimprägnierung in einem oder in zwei Schritten auf die Formkörper aufgebracht. Bei der Sprühimprägnierung wird die wässrige Imprägnierlösung auf die Trägerkörper gesprüht. Bei der Vakuumimprägnierung wird in einem mit den Formkörpern gefüllten Behälter mittels einer Vakuumpumpe ein Unterdruck erzeugt. Durch Öffnen einer Schlauchverbindung zur wässrigen Imprägnierlösung wird die Lösung in den Behälter gesaugt, bis die gesamte Schüttung der Formkörper mit der Lösung bedeckt ist. Nach einer Imprägnierzeit von 0,2 bis 2 Stunden wird die nicht durch das Material aufgenommene Lösung abgelassen oder abgegossen.

Durch Vortrocknung für die Dauer von 1 bis 10 Stunden bei Raumtemperatur kann sich das anfängliche Konzentrationsgefälle über den Querschnitt der Formkörper weitgehend ausgleichen. Somit wird die Gleichmäßigkeit der Imprägnierung über den Querschnitt der Katalysatorpartikel verbessert. Bevorzugt werden die so erhaltenen Katalysatorvorstufen für die Dauer von 1 bis 10 Stunden bei 100 bis 200, bevorzugt 100 bis 140°C zur Entfernung der Restfeuchte getrocknet. Dann erfolgt für die Dauer von 1 bis 20, bevorzugt 1 bis 5 Stunden eine Kalzinierung bei 300 bis 600, bevorzugt 420 bis 480°C. Dadurch wird der Promotor auf dem Aluminiumoxid fixiert und die Base der Imprägnierlösung zersetzt und ausgetrieben. Optional kann die Schüttung der Trägerkörper der Katalysatorvorstufen bei der Vortrocknung, Trocknung und Kalzinierung von einem Gasstrom durchströmt werden, was den Abtransport der Restfeuchte und der Zersetzungsgase verbessert.

Ein weiterer Gegenstand der vorliegenden Erfindung ist daher ein Verfahren zur Herstellung eines erfindungsgemäßen Schalenkatalysators oder Vollkatalysators, bei dem man folgende Verfahrensschritte durchführt:

a) Imprägnieren der Trägerkörper oder des Trägermaterials mit einer wässrigen Lösung, die eine lösliche Cäsiumverbindung und eine lösliche Wolframverbindung in dem Cs/W-Verhältnis von 0,8:1 bis < 2:1 enthält,

b) Vortrocknen der erhaltenen imprägnierten Formkörper oder des feinteiligen Trägermaterials (Katalysatorvorstufe) bei Raumtemperatur,

c) gegebenenfalls Trocknung bei 100 bis 200°C zur Entfernung des Restfeuchte,

d) abschließendes Kalzinieren für die Dauer von 1 bis 20 Stunden bei Temperaturen von 300 bis 600°C und

e) Erhalten des Trägerkatalysators oder des imprägnierten feinteiligen Trägermaterials mit einem Gehalt von 15 bis 45 Gew.-%, bevorzugt 20 bis 36 Gew.- eines Promotors der allgemeinen Zusammensetzung $Cs_xWO_y$, wobei man anschließend

f) das feinteilige imprägnierte Trägermaterial unter Zusatz bekannter Hilfsmittel suspendiert und auf einen inerten Trägerkern aufträgt oder extrudiert.

Die Imprägnierung der Formkörper kann auch mehrstufig, insbesondere zweistufig erfolgen.
In einer bevorzugten Ausführungsform enthält dann die in der ersten Stufe eingesetzte Lösung ein bis zu zwei Drittel der vorgesehenen Gesamtmenge an Cäsium- und Wolframverbindungen.
Geht man mehrstufig, mindestens aber zweistufig vor, kalziniert man die im ersten Schritt erhaltene Vorstufe gegebenenfalls nicht.
Ansonsten läuft in der zweiten Stufe dasselbe Imprägnier-, Trocknungs- und Kalzinierungsprogramm wie für das einstufige Verfahren beschrieben ab.
Diese mehrstufige Imprägnierung ist vor allem dann sinnvoll, wenn hohe Beladungen gewünscht werden und/oder die begrenzte Löslichkeit der Promotormischung die Beladung in einem Schritt nicht ermöglichen.
Es besteht auch die Möglichkeit, die Trägerkörper während des Imprägniervorgangs (Schritt a aus Anspruch 11) mehrfach mit der Imprägnierlösung zu besprühen und zwischen diesen Behandlungsschritten jeweils Teile der Restfeuchte bei einer Temperatur von bis zu 120°C zu entfernen, bevor man zu Schritt b übergeht.
Bei der Herstellung des Schalenkatalysators kann das als Schale aufzubringende Pulver vor oder nach der Beschichtung kalziniert werden. Beispielsweise kann dieser Katalysatortyp gemäß EP-B-0 068 193 hergestellt werden. Auch bei der Herstellung der Extrudate oder der Preßlinge kann die Kalzinierung vor und/oder nach der Verformung erfolgen.
Für die folgenden Beispiele wurden die in Tabelle 1 aufgelisteten verschiedenen Sorten an käuflichem Aluminiumoxid eingesetzt.

Tabelle 1

| Eigenschaften des verwendeten Aluminiumoxids | | |
|---|---|---|
| | Aluminiumoxid I | Aluminiumoxid II |
| Hersteller | Rhodia | Alcoa |
| Typ | Spheralite 501A | LD 350 |
| spezifische Oberfläche [m$^2$/g] | 310 | 350 |
| Schüttdichte [kg/m$^3$] | 690 - 790 | 590 - 660 |
| Wasseraufnahme [ml/g] | 0,50 | 0,58 |
| Korndurchmesser [mm] | 2 - 5 | 1,2 - 2,4 |
| Glühverlust bis 850°C [Gew.-%] | 4,5 | 5,6 |

Beispiele

Vergleichsbeispiel 1

150 g Aluminiumoxid I wurde mit 21,0 Gew.-% Cäsiumwolframat ($Cs_{2,0}WO_4$) mit Hilfe der Vakuumimprägnierung imprägniert. Hierzu wurde im einzelnen wie folgt vorgegangen:

Zur Herstellung der Imprägnierlösung wurden 55,7 g Wolframsäure in 44,5 g Wasser suspendiert und durch Zugabe von 111,4 g 25%-iger Ammoniaklösung und Erwärmen auf 50°C gelöst. 74,6 g Cs(OH)·$H_2O$ wurden in 37,3 g Wasser gelöst und mit der ersten Lösung vermischt. Die Lösung wurde anschließend im abgedeckten Becherglas 48 Stunden gerührt. Daraufhin wurde die Lösung mit 25 g Wasser auf ein Volumen von 234 ml aufgefüllt.

Das Aluminiumoxid wurde in einem Glasgefäß, das auf 150 mbar evakuiert wurde, vorgelegt. Durch das Öffnen eines Hahns wurde die Imprägnierlösung so lange in das evakuierte Glasgefäß gesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 79 ml Imprägnierlösung aufgenommen.

Das Granulat wurde für die Dauer von 1 Stunde bei Raumtemperatur im Luftstrom und anschließend bei 120°C für 3 Stunden zur Entfernung der Restfeuchte getrocknet. Danach wurde das Granulat 3 Stunden lang bei 455°C kalziniert.

Vergleichsbeispiel 2

Vergleichsbeispiel 1 wurde mit 26,3%-iger Beladung des Aluminiumoxids mit Cäsiumwolframat ($Cs_{2,0}WO_4$) wiederholt.

Beispiel 1

150 g Aluminiumoxid I wurde mit 23,4 Gew.-% Promotor ($Cs_{1,6}WO_y$) mit Hilfe der Vakuumimprägnierung imprägniert.

Hierzu wurden 71,9 g Wolframsäure in 44,5 g Wasser suspendiert und durch Zugabe von 111,4 g 25%-iger Ammoniaklösung und Erwärmen auf 50°C gelöst. 76,9 g Cs(OH)·$H_2O$ wurden in 37,3 g Wasser gelöst und mit der ersten Lösung vermischt. Die Lösung wurde anschließend im abgedeckten Becherglas 48 Stunden gerührt. Daraufhin wurde die Lösung mit 19 g Wasser auf ein Volumen von 234 ml aufgefüllt.

Imprägnierung, Trocknung und Kalzinierung wurden wie in vorangegangenen Beispielen vorgenommen.

Beispiel 2

150 g Aluminiumoxid I wurde mit 25,8 Gew.-% Promotor ($Cs_{1,3}WO_y$) mit Hilfe der Vakuumimprägnierung imprägniert.

Hierzu wurden 88,9 g Wolframsäure in 44,5 g Wasser und 177,7 g 25%-iger Ammoniaklösung gelöst. Anschließend wurden 79,3 g Cs(OH)·$H_2O$ zugegeben und die Lösung auf 50°C erwärmt. Die Lösung wurde anschließend im abgedeckten Becherglas 67 Stunden gerührt. Daraufhin wurde die Lösung mit 5 g Wasser auf ein Volumen von 234 ml aufgefüllt.

Imprägnierung, Trocknung und Kalzinierung wurden wie in vorangegangenen Beispielen vorgenommen.

Beispiel 3

150 g Aluminiumoxid I wurde in einer zweistufigen Imprägnierung mit insgesamt 30,5 Gew.-% Promotor ($Cs_{1,0}WO_y$) mit Hilfe der Vakuumimprägnierung imprägniert. Im einzelnen wurde wie folgt vorgegangen:

63,3 g Wolframsäure wurden in 50,7 g Wasser suspendiert und durch Zugabe von 126,5 g 25%-iger Ammoniaklösung und Erwärmen auf 50°C gelöst. 42,4 g $Cs(OH) \cdot H_2O$ wurden in 21,2 g Wasser gelöst und mit der ersten Lösung vermischt. Die Lösung wurde anschließend im abgedeckten Becherglas 66 Stunden gerührt. Daraufhin wurde die Lösung mit 40 g Wasser auf ein Volumen von 234 ml aufgefüllt. Das Aluminiumoxid wurde in einem Glasgefäß, das auf 150 mbar evakuiert wurde, vorgelegt. Durch das Öffnen eines Hahns wurde die Imprägnierlösung so lange angesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 76 ml Imprägnierlösung aufgenommen. Anschließend wurde das Granulat 1 Stunde bei Raumtemperatur und 3 Stunden bei 120°C getrocknet sowie 3 Stunden bei 455°C kalziniert. Auf den Katalysatorpartikeln hatten sich durch diese Behandlung 18,7 Gew.-% Promotor abgelagert.

Zur Durchführung der zweiten Imprägnierung wurde eine gleiche Imprägnierlösung wie beim ersten Schritt angesetzt und in gleicher Weise durch Vakuumimprägnierung auf den bereits beladenen Katalysator aus dem ersten Schritt aufgebracht. Dann schloss sich wieder eine 1-stündige Trocknung bei Raumtemperatur an, gefolgt von einer 3-stündigen Trocknung bei 120°C. Abschließend wurden die Katalysatorpartikel 4 Stunden bei 455°C an Luft kalziniert.

Beispiel 4

Beispiel 3 wurde mit 31,4%-iger Beladung des Aluminiumoxids mit Cäsiumwolframat ($Cs_{1,4}WO_y$) wiederholt.

Beispiel 5

Beispiel 3 wurde mit 34,5%-iger Beladung des Aluminiumoxids mit Cäsiumwolframat ($CS_{1,6}WO_y$) wiederholt.

Beispiel 6

Beispiel 3 wurde mit 33,2%-iger Beladung des Aluminiumoxids mit Cäsiumwolframat ($Cs_{1,4}WO_y$) wiederholt. Anstelle des Aluminiumoxids I wurde jedoch das Aluminiumoxid II eingesetzt.

Beispiel 7

300 g Aluminiumoxid I wurde mit insgesamt 35,3 Gew.-% Promotor ($Cs_{1,4}WO_y$) mittels Sprühimprägnierung imprägniert.

Zur Herstellung der Imprägnierlösung wurden 95,1 g Wolframsäure in 76,0 g Wasser suspendiert und durch Zugabe von 190,1 g 25%-iger Ammoniaklösung und Erwärmen auf 50°C gelöst. 90,2 g $CsOH \cdot H_2O$ wurden in 45,1 g Wasser gelöst und mit der ersten Lösung vermischt. Die Lösung wurde anschließend im abgedeckten Becherglas 48 Stunden gerührt. Das in einem Dragierkessel umgewälzte Granulat wurde mit der Imprägnierlösung besprüht. Da das Granulat aufgrund der begrenzten Wasseraufnahme nicht das gesamte Flüssigkeitsvolumen der Imprägnierlösung aufnehmen konnte, wurde das Granulat zwischen dem Besprühen mit der Imprägnierung mehrmals mit einem Heißluftfön auf 110°C erwärmt, um Teile der Restfeuchte zu entfernen.

Das Granulat wurde dann für die Dauer von 1 Stunde an Luft gelagert und anschließend bei 120°C für 3 Stunden zur Entfernung der Restfeuchte getrocknet. Danach wurde das Granulat 3 Stunden lang bei 455°C kalziniert.

Beispiel 8

150 g Aluminiumoxid I wurde mit 31,1 Gew.-% Promotor ($Cs_{1,4}WO_y$) mit Hilfe der Vakuumimprägnierung imprägniert.

Hierzu wurden 123,0 g Wolframsäure in 230,0 g 25%-iger Ammoniaklösung suspendiert sowie 116,2 g $CsOH \cdot H_2O$ zugegeben. Sofort nach dem Erwärmen auf 50°C wurde die noch heiße Imprägnierlösung wie in Vergleichsbeispiel 1 beschrieben mittels Vakuumimprägnierung auf das Aluminiumoxid aufgebracht.

Trocknung und Kalzinierung wurden wie in vorangegangenen Beispielen vorgenommen.

Anwendungsbeispiel

Die Katalysatoren wurden bezüglich ihrer Leistungsdaten bei der Synthese von Methylmercaptan aus Schwefelwasserstoff und Methanol getestet.

Die Synthese wurde in einem Edelstahlrohr von 18 mm Innendurchmesser und einer Länge von 500 mm durchgeführt. Die Katalysatorschüttung von jeweils 76 ml wurde beiderseits durch Inertschüttungen aus Glaskugeln im Reaktionsrohr fixiert. Das Reaktionsrohr wurde über einen Doppelmantel mit einem Thermoöl auf die Reaktionstemperatur von etwa 320°C aufgeheizt.

Die Versuchsbedingungen sind der folgenden Aufstellung zu entnehmen:

| | |
|---|---|
| GHSV: | 1300 h$^{-1}$ (bezogen auf Normbedingungen) |
| LHSV: | 0,84 h$^{-1}$ (bezogen auf flüssiges MeOH) |
| Reaktionstemperatur: | 320°C |
| Massenverhältnis $H_2S$/MeOH: | 1,9 |
| Druck: | 9 bar |

Das Reaktionsgemisch mit den Produkten Methylmercaptan, Dimethylsulfid und Dimethylether und mit den nicht umgesetzten Eingangsstoffen Methanol und Schwefelwasserstoff wird durch online Gaschromatographie analysiert.

Die Messergebnisse sind Tabelle 2 zu entnehmen. Wie die Ergebnisse zeigen, verbessert die Erhöhung der Cäsiumwolframat ($Cs_{2,0}WO_4$)-Beladung die Selektivität, jedoch verschlechtern sich dadurch die Aktivität und die Ausbeute (Vergleichsbeispiele 1 und 2).

Wird der Wolframanteil im Verhältnis zum Cäsiumanteil im Katalysator erhöht, so ist eine deutliche Steigerung der Aktivität bei gleichzeitig verbesserter Selektivität zu erkennen. Dies führt im Vergleich zum Stand der Technik zu einer Ausbeutesteigerung von bis zu 13%. Die Selektivität kann durch Erhöhung des Wolframgehalts und durch Erhöhung der Gesamtbeladung auf über 30 Gew.-% bis auf ~ 97% gesteigert werden, wobei der Methanolumsatz ansteigt. Dies führt bei der großtechnischen Synthese von Methylmercaptan auch zu erheblichen Kosteneinsparungen bei der Abtrennung des Reaktionsproduktes von nicht umgesetzten Methanol und Nebenprodukten.

**Tabelle 2** - Versuchsergebnisse

| Katalysator | Aluminium-oxid | mol-Verhält. Cs:W | Beladung [Gew.-%] | Methanolumsatz [%] | Selektivität [%] | Ausbeute [%] |
|---|---|---|---|---|---|---|
| VB1 | I | 2:1 | 21,0 | 82,4 | 93,3 | 76,9 |
| VB2 | I | 2:1 | 26,3 | 79,5 | 94,7 | 75,2 |
| B1 | I | 1,6:1 | 23,4 | 85,0 | 94,9 | 80,7 |
| B2 | I | 1,3:1 | 25,8 | 90,1 | 94,9 | 85,5 |
| B3*) | I | 1,0:1 | 30,5 | 97,6 | 92,0 | 89,8 |
| B4*) | I | 1,4:1 | 31,4 | 89,1 | 96,1 | 85,6 |
| B5*) | I | 1,6:1 | 34,5 | 85,1 | 97,0 | 82,5 |
| B6*) | II | 1,6:1 | 33,2 | 91,6 | 96,9 | 88,8 |
| B7*) | I | 1,4:1 | 35,5 | 85,9 | 95,9 | 82,4 |
| B8 | I | 1,4:1 | 31,1 | 88,6 | 96,2 | 85,2 |
| VB1: Katalysator nach Vergleichsbeispiel 1 *) mehrstufige Imprägnierung | | | | | | |

**Patentansprüche**

1. Trägerkatalysator, enthaltend eine katalytisch aktive oxidische Zusammensetzung der allgemeinen Formel $Cs_xWO_y$, in der bedeuten

$$x: 0,8 \text{ bis} < 2$$

und

$$y: 3,4 \text{ bis} < 4,$$

wobei der Trägerkatalysator die oxidische Zusammensetzung in einer Menge von 15 bis 45 Gew.-% enthält, und wobei ein Trägerkörper oder ein pulverförmiger Träger mit einer wässrigen Imprägnierlösung aus in Wasser löslichen Cs- und Wolframverbindungen imprägniert und abschließend kalziniert wird.

2. Schalenkatalysator, bei dem ein Trägerkern mit einem pulverförmigen Trägerkatalysator gemäß Anspruch 1 umhüllt ist.

3. Vollkatalysator bei dem ein pulverförmiger Trächterkatalysator gemäß Anspruch 1 verformt wird.

4. Vollkatalysator gemäß Anspruch 3, **dadurch gekennzeichnet, dass** sich das Verhältnis von Cäsium zu Wolfram auf 1,9:1 bis 1:1 beläuft.

5. Katalysator gemäß einem oder mehreren der Ansprüche 1 bis 4, **dadurch gekennzeichnet, dass** er die oxidische Zusammensetzung in einer Menge von 20 bis 36 Gew.-% enthält, wobei im Falle eines Schalenkatalysators sich diese Anteile auf die Zusammensetzung der Schale beziehen.

6. Katalysator gemäß einem oder mehreren der Ansprüche 1 bis 5, **dadurch gekennzeichnet, dass** der Trägerkörper oder das Trägermaterial aus einer oxidischen anorganischen Verbindung besteht.

7. Katalysator gemäß Anspruch 6, **dadurch gekennzeichnet, dass** der Trägerkörper oder das Trägermaterial aus Aluminiumoxid ($Al_2O_3$) besteht.

8. Katalysator gemäß Anspruch 7, **dadurch gekennzeichnet, dass** der Trägerkörper eine spezifische Oberfläche von 180 bis 400 m$^2$/g (BET) und ein Gesamtporenvolumen von 0,3 bis 1,2 ml/g hat.

9. Verfahren zur Herstellung eines Katalysators gemäß den Ansprüchen 2 und 3, bei dem man folgende Verfahrensschritte durchführt:

    a) Imprägnieren der Trägerkörper oder des Trägermaterials mit einer wässrigen Lösung, die eine lösliche Cäsiumverbindung und eine lösliche Wolframverbindung in dem Cs/W-Mol-Verhältnis von 0,8:1 bis < 2:1 enthält,
    b) Vortrocknen der erhaltenen imprägnierten Formkörper oder des feinteiligen Trägermaterials (Katalysatorvorstufe) bei Raumtemperatur,
    c) gegebenenfalls Trocknung bei 100 bis 200°C zur Entfernung der Restfeuchte,
    d) abschließendes Kalzinieren für die Dauer von 1 bis 20 Stunden bei Temperaturen von 300 bis 600°C und
    e) Erhalten des Trägerkatalysators oder des imprägnierten feinteiligen Trägermaterials mit einem Gehalt von 15 bis 45 Gew.-%, bevorzugt 20 bis 36 Gew.-% eines Promotors der allgemeinen Zusammensetzung Cs$_x$WO$_y$, wobei man anschließend
    f) das feinteilige imprägnierte Trägermaterial unter Zusatz bekannter Hilfsmittel suspendiert und auf einen inerten Trägerkern aufträgt oder extrudiert und verpresst.

10. Verfahren gemäß Anspruch 9, **dadurch gekennzeichnet, dass** man die Schritte a bis c und gegebenenfalls d mindestens einmal wiederholt.

11. Verfahren gemäß Anspruch 10, **dadurch gekennzeichnet, dass** bei mehrmaligem Imprägnieren die zuerst eingesetzte Imprägnierlösung ein bis zwei Drittel der vorgesehenen Gesamtmenge an Cäsium und Wolfram enthält.

12. Verfahren gemäß den Ansprüchen 10 und 11, **dadurch gekennzeichnet, dass** man die Trägerkörper oder das Trägermaterial mehrfach mit der Imprägnierlösung besprüht und zwischen diesen Behandlungsschritten bei einer Temperatur bis 120°C Teile der Restfeuchte entfernt, bevor man zu dem Behandlungsschritt b) übergeht.

**13.** Verfahren gemäß Anspruch 9, **dadurch gekennzeichnet, dass** man den Katalysator nach dem Auftragen des imprägnierten Trägermaterials auf den Kern nach der Extrusion oder Verpressung tempert.

**14.** Verfahren zur Herstellung von Alkylmercaptanen durch Umsetzung von Alkanolen und Schwefelwasserstoff in Gegenwart des Katalysators gemäß den Ansprüchen 1 bis 8.

**15.** Verfahren gemäß Anspruch 14 zur Herstellung von Methylmercaptan durch Umsetzung von Methylalkohol und Schwefelwasserstoff.

**Claims**

**1.** Supported catalyst containing a catalytically active oxidic composition of the general formula $Cs_xWO_y$, where the indices have the following meanings

$$x: 0.8 \text{ to} < 2$$

and

$$y: 3.4 \text{ to} < 4,$$

wherein the supported catalyst contains the oxidic composition in an amount of from 15 to 45% by weight and wherein a support body or a pulverulent support is impregnated with an aqueous impregnation solution of water-soluble Cs compounds and tungsten compounds and finally calcined.

**2.** Shell catalyst in which a support core is coated with a pulverulent supported catalyst according to Claim 1.

**3.** Solid catalyst in which a pulverulent supported catalyst according to Claim 1 is shaped.

**4.** Solid catalyst according to Claim 3, **characterized in that** the ratio of caesium to tungsten is from 1.9:1 to 1:1.

**5.** Catalyst according to one or more of Claims 1 to 4, **characterized in that** it contains the oxidic composition in an amount of from 20 to 36% by weight, where in the case of a shell catalyst these proportions relate to the composition of the shell.

**6.** Catalyst according to one or more of Claims 1 to 5, **characterized in that** the support body or the support material consists of an oxidic inorganic compound.

**7.** Catalyst according to Claim 6, **characterized in that** the support body or the support material consists of aluminium oxide ($Al_2O_3$).

**8.** Catalyst according to Claim 7, **characterized in that** the support body has a specific surface area of from 180 to 400 $m^2$/g (BET) and a total pore volume of from 0.3 to 1.2 ml/g.

**9.** Process for producing a catalyst according to Claims 2 and 3, wherein the following process steps are carried out:

a) impregnation of the support bodies or of the support material with an aqueous solution which contains a soluble caesium compound and a soluble tungsten compound in a Cs/W molar ratio of from 0.8:1 to < 2:1,
b) predrying of the obtained impregnated shaped bodies or of the finely divided support material (catalyst precursor) at room temperature,
c) optionally drying at from 100 to 200°C to remove the residual moisture,
d) final calcination for a time of from 1 to 20 hours at temperatures of from 300 to 600°C and
e) obtaining the supported catalyst or of the impregnated finely divided support material having a content of from 15 to 45% by weight, preferably from 20 to 36% by weight, of a promoter of the general composition $Cs_xWO_y$, where subsequently

**EP 1 656 343 B2**

f) the finely divided impregnated support material is suspended with addition of known auxiliaries and applied to an inert support core or extruded and pressed.

10. Process according to Claim 9, **characterized in that** the steps a to c and optionally d are repeated at least once.

11. Process according to Claim 10, **characterized in that** in the case of multiple impregnation, the impregnation solution used first contains from one to two thirds of the total amount of caesium and tungsten provided.

12. Process according to Claims 10 and 11, **characterized in that** the support bodies or the support material are sprayed a plurality of times with the impregnation solution and parts of the residual moisture are removed at a temperature of up to 120°C between these treatment steps before proceeding to the treatment step b).

13. Process according to Claim 9, **characterized in that** the catalyst is tempered after application of the impregnated support material onto the core after extrusion or pressing.

14. Process for preparing alkyl mercaptans by reaction of alkanols and hydrogen sulphide in the presence of the catalyst according to any of Claims 1 to 8.

15. Process according to Claim 14 for preparing methyl mercaptan by reaction of methyl alcohol and hydrogen sulphide.

**Revendications**

1. Catalyseur supporté, contenant une composition oxydique catalytiquement active de formule générale $Cs_xWO_y$, dans laquelle

$$x \text{ signifie : } 0,8 \text{ à} < 2$$

et

$$y \text{ signifie : } 3,4 \text{ à} < 4,$$

le catalyseur supporté contenant la composition oxydique dans une quantité de 15 à 45 % en poids et un corps support ou un support pulvérulent étant imprégné avec une solution d'imprégnation aqueuse constituée de composés de Cs et de tungstène solubles dans l'eau et ensuite calciné.

2. Catalyseur à coquille, dans lequel un noyau support est enrobé d'un catalyseur supporté pulvérulent selon la revendication 1.

3. Catalyseur massique, dans lequel un catalyseur supporté pulvérulent selon la revendication 1 est déformé.

4. Catalyseur massique selon la revendication 3, **caractérisé en ce que** le rapport entre le césium et le tungstène est de 1,9:1 à 1:1.

5. Catalyseur selon une ou plusieurs des revendications 1 à 4, **caractérisé en ce qu'**il contient la composition oxydique en une quantité de 20 à 36 % en poids, ces proportions se rapportant, dans le cas d'un catalyseur à coquille, à la composition de la coquille.

6. Catalyseur selon une ou plusieurs des revendications 1 à 5, **caractérisé en ce que** le corps support ou le matériau de support est constitué d'un composé inorganique oxydique.

7. Catalyseur selon la revendication 6, **caractérisé en ce que** le corps support ou le matériau de support est constitué d'oxyde d'aluminium ($Al_2O_3$).

8. Catalyseur selon la revendication 7, **caractérisé en ce que** le corps support a une surface spécifique de 180 à 400

m$^2$/g (BET) et un volume poreux total de 0,3 à 1,2 ml/g.

9. Procédé de fabrication d'un catalyseur selon les revendications 2 et 3, dans lequel les étapes de procédé suivantes sont réalisées :

a) l'imprégnation du corps support ou du matériau de support avec une solution aqueuse qui contient un composé de césium soluble et un composé de tungstène soluble dans un rapport molaire Cs/W de 0,8:1 à < 2:1,
b) le pré-séchage du corps moulé imprégné ou du matériau de support finement divisé imprégné obtenu (précurseur de catalyseur) à température ambiante,
c) éventuellement le séchage à 100 à 200 °C pour éliminer l'humidité résiduelle,
d) enfin la calcination pendant une durée de 1 à 20 heures à des températures de 300 à 600 °C, et
e) l'obtention du catalyseur supporté ou du matériau de support finement divisé imprégné ayant une teneur de 15 à 45 % en poids, de préférence de 20 à 36 % en poids en promoteur de composition générale Cs$_x$WO$_y$,
f) le matériau de support imprégné finement divisé étant ensuite suspendu avec addition d'adjuvants connus et appliqué sur un noyau support inerte ou extrudé et comprimé.

10. Procédé selon la revendication 9, **caractérisé en ce que** les étapes a à c et éventuellement d sont répétées au moins une fois.

11. Procédé selon la revendication 10, **caractérisé en ce qu'**en cas d'imprégnation multiple, la solution d'imprégnation utilisée en premier contient un à deux tiers de la quantité totale prévue de césium et de tungstène.

12. Procédé selon les revendications 10 et 11, **caractérisé en ce que** le corps support ou le matériau de support est pulvérisé à plusieurs reprises avec la solution d'imprégnation et des parties de l'humidité résiduelle sont éliminées entre ces étapes de traitement à une température de jusqu' à 120 °C, avant de passer à l'étape de traitement b).

13. Procédé selon la revendication 9, **caractérisé en ce que** le catalyseur est recuit après l'application du matériau de support imprégné sur le noyau après l'extrusion ou la compression.

14. Procédé de fabrication d'alkylmercaptans par mise en réaction d'alcanols et de sulfure d'hydrogène en présence du catalyseur selon les revendications 1 à 8.

15. Procédé selon la revendication 14 pour la fabrication de méthylmercaptan par mise en réaction d'alcool méthylique et de sulfure d'hydrogène.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2820062 A
- EP 0832687 B1
- EP 0068193 B

**In der Beschreibung aufgeführte Nicht-Patentliteratur**

- **S. LARUELLE et al.** *Journal of Solid State Chemistry,* 1994, vol. 111, 172-177
- **T. KUDO.** *Solid State Ionics,* 1994, vol. 72, 204-208
- **Q. ZHONG et al.** *Thin Solid Films,* 1991, vol. 205, 85-88
- **A.V. MASHKINA et al.** *React. Kinet. Catal. Lett.,* 1988, vol. 36 (1), 159-164
- Ullmann's Enzyclopedia of Industrial Chemistry. 1985, vol. A1, 561-562